# EUROPEAN PATENT APPLICATION

(11) **EP 2 614 819 A1**
(43) Date of publication of application: **17.07.2013**
(21) Application number: 11821346.1
(22) Date of filing: 01.09.2011
(51) Int. Cl.: A61K 9/70, A61K 47/06, A61K 47/32

(54) **PERCUTANEOUS ABSORBENT AND ADHESIVE SHEET FOR SKIN PATCH**

(30) Priority: 03.09.2010 JP 2010198335
(71) Applicant: KM Transderm, Osaka 530-0005 (JP); Medrx Co., Ltd., Kagawa 769-2712 (JP)
(72) Inventor: AKAZAWA, Mitsuji, Higashikagawa-shi Kagawa 769-2702 (JP); YAMASAKI, Keiko, Higashikagawa-shi Kagawa 769-2712 (JP)
(74) Representative: Duckworth, Timothy John
(86) International application number: PCT/JP2011/004922
(87) International publication number: WO 2012/029325

(57) **Abstract**

Provided is a transdermal absorption preparation showing low skin irritation and sufficient drug releaseability, and further, an adhesive sheet for adhesion to the skin, which has sufficient adhesiveness and causes low skin irritation. Moreover, provided is a transdermal absorption preparation having, in addition to the above-mentioned property, sufficient drug releaseability.

An adhesive sheet for adhesion to the skin, which comprises a support and an adhesive layer formed on the support, wherein the adhesive layer contains at least a thermoplastic elastomer, and more than 300 parts by weight of liquid paraffin per 100 parts by weight of the elastomer, and wherein the adhesive layer contains not more than 10 wt% of a tackifier.

## Description

### Technical Field

The present invention relates to an adhesive sheet for adhesion to the skin, which has sufficient adhesiveness and causes low skin irritation, and a transdermal absorption preparation showing low skin irritation and improved drug releaseability.

### Background Art

When a drug is to be transdermally absorbed, the drug is added to an adhesive base and the like and formed as an adhesive preparation. In recent years, tapes more superior in the adhesiveness are often used than poultices containing a large amount of water as a constituent component in an adhesive preparation. As an adhesive base for such tapes, a lipophilic adhesive base such as of rubber, acrylic or silicon type and the like is used. Of these, a rubber adhesive base is widely used since additives can be easily blended as compared to other adhesive bases. (patent documents 1 - 3)
However, problems have been pointed out even for a transdermal absorption preparation using a rubber adhesive base such as unensurable sufficient releaseability of a drug, development of skin irritation caused by a tackifier generally added to a transdermal absorption preparation and the like.

### [Document List]

### [patent documents]

[patent document 1]JP-A-2001-302502
[patent document 2]JP-A-9-291028
[patent document 3]JP-A-10-316559
[non-patent document]

[non-patent document 1]"Nenchaku seihin no oyo gijutsu (Applied technology of adhesive product)" pp. 125 - 141, Keiji Fukuzawa et al., CMC Publishing Co., Ltd., 2000

### SUMMARY OF THE INVENTION

### Problems to be Solved by the Invention

An object of the present invention is to provide an adhesive sheet for adhesion to the skin, which has sufficient adhesiveness and causes low skin irritation, and a transdermal absorption preparation showing low skin irritation and sufficient drug releaseability.

### Means of Solving the Problems

The present inventors have conducted intensive studies in an attempt to solve the aforementioned problems and found that an adhesive sheet for adhesion to the skin, which has sufficient adhesiveness and causes low skin irritation, can be obtained even without using a tackifier by using, as an adhesive base, a thermoplastic elastomer and a large amount of liquid paraffin relative to the elastomer. They have further found that a transdermal absorption preparation having sufficient transdermal absorbability can be obtained by adding a drug or a pharmaceutically acceptable salt thereof to the adhesive sheet, which resulted in the present invention.

Accordingly, the gist of the present invention is as follows.
(1) An adhesive sheet for adhesion to the skin, which comprises a support and an adhesive layer formed on the support, wherein the aforementioned adhesive layer comprises at least a thermoplastic elastomer, and more than 300 parts by weight of liquid paraffin per 100 parts by weight of the elastomer, and wherein the adhesive layer comprises not more than 10 wt% of a tackifier.
(2) The adhesive sheet of the above-mentioned (1), wherein the content of the liquid paraffin in the adhesive layer is not less than 60 wt%.
(3) The adhesive sheet of the above-mentioned (1) or (2), wherein the thermoplastic elastomer is a styrene-based block copolymer.
(4) The adhesive sheet of the above-mentioned (3), wherein the styrene-based block copolymer is a styrene-isoprene-styrene copolymer.
(5) The adhesive sheet of any of the above-mentioned (1) - (4), wherein the adhesive layer does not contain a tackifier.
(6) A transdermal absorption preparation comprising the adhesive sheet of any of the above-mentioned (1) - (5) and a drug or a pharmaceutically acceptable salt thereof in the adhesive layer of the sheet.

### Effect of the Invention

The adhesive sheet for adhesion to the skin of the present invention has sufficient adhesiveness and causes low skin irritation when adhered to the skin. In addition, the transdermal absorption preparation of the present invention also shows good drug releaseability.

### Brief Description of the Drawings

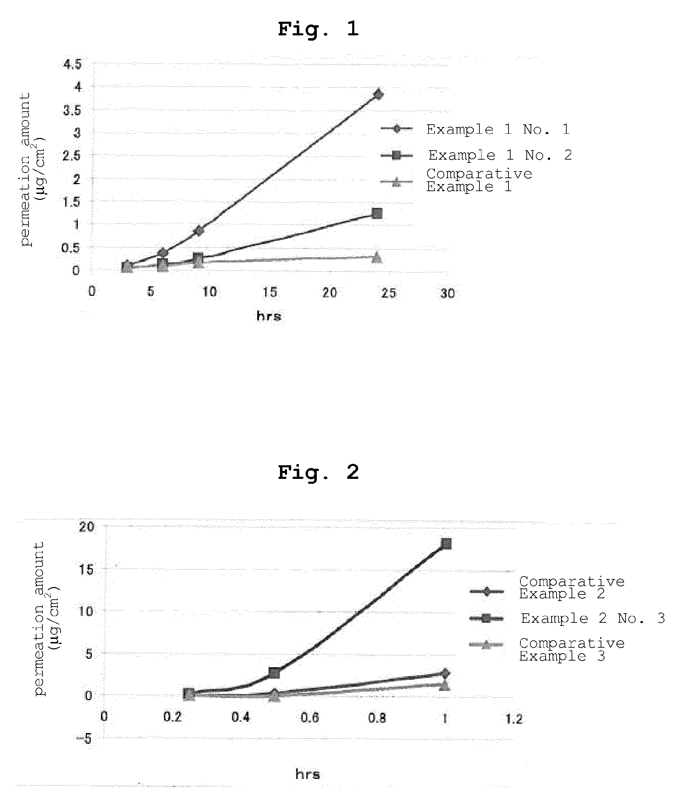

Fig. 1 is a chart showing the comparison of the transdermal absorbability between the adhesive preparations of test No. 1 and test No. 2 of Example 1 of the present invention, and that of Comparative Example 1.
Fig. 2 is a chart showing the comparison of the transdermal absorbability among the adhesive preparations of Example 2 of Example 2 of the present invention, and those of Comparative Example 2 (commercially available lidocaine-containing adhesive preparation: containing 60 w/w% lidocaine), and Comparative Example 3 (commercially available lidocaine-containing adhesive preparation: containing 10 w/w% lidocaine). Description of Embodiments

The adhesive sheet for adhesion to the skin and the transdermal absorption preparation of the present invention characteristically have an adhesive layer formed on a support, wherein the adhesive layer contains at least a thermoplastic elastomer, and more than 300 parts by weight of liquid paraffin per 100 parts by weight of the elastomer, and not more than 10 wt% of a tackifier in the adhesive layer.
The "thermoplastic elastomer" in the present invention is a thermoplastic elastomer having a hard segment and a soft segment, and examples thereof include various thermoplastic elastomers of urethane-based, acrylic, styrene-based or olefin-based type and the like. Of these, a styrene-based thermoplastic elastomer, particularly, a styrene-based block copolymer is preferably used for simultaneous achievement of sufficient adhesiveness and low skin irritation, which is the object of the present invention.
Specific examples include styrene-butadiene block copolymer, styrene-butadiene-styrene block copolymer, styrene-isoprene block copolymer, styrene-isoprene-styrene block copolymer, styrene-ethylene/butylene block copolymer, styrene-ethylene/butylene-styrene block copolymer, styrene-ethylene/propylene block copolymer, styrene-ethylene/propylene-styrene block copolymer, styrene-isobutylene block copolymer, styrene-isobutylene-styrene block copolymer and the like. Of these, only one kind of a styrene-based block copolymer may be used, or two or more kinds thereof may be used in combination.
Among these styrene-based block copolymers, a styrene-isoprene-styrene block copolymer, a styrene-isoprene block copolymer, and a mixture thereof are particularly preferably used to simultaneously achieve sufficient adhesiveness and low skin irritation, and in view of the availability and handling property for adhesion to the skin.

The "liquid paraffin" of the present invention is not particularly limited and a known commercially available product can be used.
As mentioned above, more than 300 parts by weight of liquid paraffin is added per 100 parts by weight of the thermoplastic elastomer in the present invention. As long as this ratio is satisfied, specific amounts of the thermoplastic elastomer and the liquid paraffin in the adhesive layer are not particularly limited. Generally, when the amount of the thermoplastic elastomer is too small, the form of an adhesive is difficult to maintain, and when it is too much, sufficient adhesiveness cannot be achieved. On the other hand, when the amount of the liquid paraffin is too small, sufficient adhesiveness is not obtained, and when it is too much, the form of an adhesive is difficult to maintain.
The lower limit of the thermoplastic elastomer content is generally 5 wt%, preferably 8 wt%, more preferably 10 wt%. The upper limit is generally 25 wt%, preferably 20 wt%. The lower limit of the liquid paraffin content is generally 60 wt%, preferably 65 wt%, more preferably 70 wt%, particularly preferably 75 wt%. The upper limit is generally 95 wt%, preferably 90 wt%.

The adhesive sheet of the present invention can exhibit good adhesiveness even when a tackifier is not added, by adopting the above-mentioned amounts of the thermoplastic elastomer and the liquid paraffin. The "tackifier" here means tackifiers generally used widely in the field of adhesive preparation. Examples thereof include rosin-based resin, polyterpene resin, cumarone-indene resin, petroleum-based resin, terpene-phenol resin, alicyclic saturated hydrocarbon resin and the like.
In the present invention, the content of the tackifier in the adhesive layer is not more than 10 wt% to reduce skin irritation and the like. It is preferably not more than 5 wt%, more preferably not more than 2 wt%, still more preferably not more than 1 wt%. Most preferably, no tackifier is contained.
In the present invention, a drug or a pharmaceutically acceptable salt thereof is further added to the adhesive layer of an adhesive sheet for adhesion to the skin to give a transdermal absorption preparation.

The "drug or a pharmaceutically acceptable salt thereof" in the present invention refers to a drug or a salt thereof to be used for transdermal absorption, and is not particularly limited. Examples thereof include anti-inflammatory agents such as acetaminophen, phenacetin, mefenamic acid, diclofenac sodium, flufenamic acid, aspirin, sodium salicylate, methyl salicylate, glycol salicylate, aminopyrine, alclofenac, ibuprofen, naproxen, flurbiprofen, ketoprofen, amfenac sodium, mepirizole, indomethacin, piroxicam, felbinac and the like; steroidal anti-inflammatory drugs such as hydrocortisone, triamcinolone, dexamethasone, prednisolone and the like; vasodilators such as diltiazem hydrochloride, pentaerythritol tetranitrate, isosorbide nitrate, tradipil, nicorandil, nitroglycerol, prenylamine lactate, molsidomine, amyl nitrite, tolazoline hydrochloride, nifedipine and the like; antiarrhythmic agents such as procaineamide hydrochloride, lidocaine hydrochloride, propranolol hydrochloride, alprenolol hydrochloride, atenolol, nadolol, metoprolol tartrate, ajmaline, disopyramide, mexiletine hydrochloride and the like; antihypertensive agents such as ecarazine hydrochloride, indapamide, clonidine hydrochloride, bunitrolol hydrochloride, labetalol hydrochloride, captopril, guanabenz acetate, mebutamate, bethanidine sulfate and the like; antitussive expectorants such as carbetapentane citrate, cloperastine, oxeladin tannate, cloputinol hydrochloride, clofedanol hydrochloride, noscapine hydrochloride, ephedrine hydrochloride, isoproterenol hydrochloride, cloriprenaline hydrochloride, methoxyphenamine hydrochloride, procaterol hydrochloride, tulobuterol hydrochloride, clenputerol hydrochloride, ketotifen fumarate and the like; antineoplastic drugs such as cyclophosphamide, fluorouracil, degafur, mitomycin C, procarbazine hydrochloride, doxifluridine, ranimustine and the like; topical anesthetics such as ethyl aminobenzoate, tetracaine hydrochloride, brocaine hydrochloride, dibucaine hydrochloride, oxybuprocaine hydrochloride, propitocaine hydrochloride and the like; hormone preparations such as propylthiouracil, thiamazole, metelonone acetate, estradiol, estriol, progesterone and the like; antihistamine agents such as diphenhydramine hydrochloride, chlorpheniramine maleate, promethazine, dyproheptadine hydrochloride, diphenylpyraline hydrochloride and the like; anticoagulants such as warfarin potassium, ticlopidine hydrochloride and the like; anticonvulsive agents such as atropine methylbromide, scopolamine and the like; general anesthetics such as thiopental sodium, pentobarbital sodium and the like; hypnotics or analgesics such as bromovalenylurea, amobarbital, phenobarbital and the like; antiepileptic agents such as phenytoin sodium and the like; analeptics or stimulant drugs such as methamphetamine hydrochloride and the like; antidizziness drugs such as difendol hydrochloride, betahistine mesilate and the like; psychoneurotic agents such as chlorpromazine hydrochloride, thioridazine, meprobamate, imipramine hydrochloride, chlordiazepoxide, diazepam and the like; muscle relaxants such as suxamethonium hydrochloride, eperisone hydrochloride and the like; autonomic agents such as neostigmine bromide, bethanechol chloride and the like; antiparkinson agents such as amantadine hydrochloride and the like; diuretics such as hydroflumethiazide, isosorbide, furosemide and the like; vasoconstrictors such as phenylephrine hydrochloride and the like; respiratory stimulants such as lobeline bromide, dimorpholamine, naloxone hydrochloride and the like; peptic ulcer therapeutic agents such as glycopyrronium bromide, proglumide, cetraxate hydrochloride, cimetidine, spizofurone and the like; cholagogues such as ursodesoxycholic acid, osalmid and the like; urogenital and anus agents such as hexamine, sparteine, dinoprost, ritodrine hydrochloride and the like; agents for parasitic skin diseases such as salicylic acid, ciclopirox olamine, coroconazole hydrochloride and the like; skin softeners such as urea and the like; vitamins such as calcitriol, thiamine hydrochloride, riboflapin sodium phosphate, pyridoxine hydrochloride, nicotinamide, panthenol, ascorbic acid and the like; mineral preparations such as calcium chloride, potassium iodide, sodium iodide and the like; hemostatic drugs such as ethamsylate drug; agents for liver diseases such as tiopronin and the like; agents for habitual intoxication such as cyanamide and the like; therapeutic agents for gout such as colchicine, probenecid, sulfinpyrazone and the like; diabetic agents such as tolbutamide, chlorpropamide, glymidine sodium, glypuzole, puformin hydrochloride, insulin and the like; antibiotics such as benzylpenicillin potassium, propicillin potassium, cloxacillin sodium, ampicillin sodium, bacampillicin hydrochloride, carbenicillin sodium, cephaloridine, cefoxitin sodium, erythromycin, chloramphenicol, tetracycline, kanamycin sulfate, cycloserine and the like; chemotherapeutic agents such as isocyanide, pyrazinamide, ethionamide and the like; narcotics such as morphine hydrochloride, codeine phosphate, cocaine hydrochloride, pethidine hydrochloride, fentanyl citrate and the like; and the like.
The adhesive sheet for adhesion to the skin and transdermal absorption preparation of the present invention are constituted by extending an adhesive layer having the above-mentioned constitution on a support.

The "support" in the present invention is not particularly limited and those used widely can be employed. For example, stretchable or non-stretchable woven fabric or non-woven fabric of polyethylene, polypropylene and the like, films of polyethylene, polypropylene, ethylene vinyl acetate copolymer, vinyl chloride and the like, foamed supports of urethane, polyurethane and the like can be mentioned. These may be used alone, or plural kinds thereof may be laminated and uses. To prevent accumulation of static electricity on the support, as well as to achieve good anchor property to the adhesive layer, a non-woven fabric or woven fabric containing an antistatic agent can be used.
Moreover, the transdermal absorption preparation of the present invention may contain excipient, antioxidant, flavor, colorant and the like as an optional component.
Examples of the excipient to be used in the present invention include silicon compounds such as silicic anhydride, light anhydrous silicic acid, hydrated silicate and the like, cellulose derivatives such as ethylcellulose, methylcellulose, hydroxypropylcellulose, hydroxypropylmethylcellulose and the like, water-soluble polymers such as polyvinyl alcohol and the like, aluminum compounds such as dried aluminum hydroxide gel, hydrated aluminum silicate and the like, kaolin, titanium oxide and the like.
Examples of the antioxidant to be used in the present invention include dibutylhydroxytoluene, ascorbic acid, tocopherol, tocopherol ester derivative, butylhydroxyanisole, 2-mercaptobenzimidazole and the like.

### Examples

The present invention is explained in more detail in the following by referring to Examples and Comparative Examples, which are not to be construed as limitative.

### (Example 1) Preparation of transdermal absorption adhesive preparation containing diclofenac

Each agent is weighed to achieve the composition (w/w%) of the following Table 1, a styrene-isoprene-styrene copolymer is added to liquid paraffin, and the mixture is dissolved by heating to about 160°C. The solution is cooled to 100°C, propylene glycol containing diclofenac hydroxyethylpyrrolidine salt is added and the mixture is mixed and stirred to give an adhesive base.
The adhesive base is applied to a silicon-treated polyester film, and adjusted to an amount of 1000 g/m². A polyester non-woven fabric is laminated on the surface of the adhesive base. This was cut in a desired size to give the object transdermal absorption adhesive preparation.

**[Table 1]**

| agent | No. 1 | No. 2 |
|---|---|---|
| thermoplastic elastomer: styrene-isoprene-styrene copolymer | 15 | 15 |
| liquid paraffin | 75 | 78.7 |
| polyhydric alcohol: propylene glycol | 5 | 5 |
| drug: diclofenac hydroxyethylpyrrolidine salt | 5 | 1.3 |

The transdermal absorption preparation prepared in the above-mentioned Table 1 showed good feeling and good adhesive property to the skin. As for transdermal absorbability, as shown in Fig. 1, even adhesive preparations having the same drug content, comparison of No. 2 of the present invention and Comparative Example 1 (commercially available adhesive preparation containing diclofenac hydroxyethylpyrrolidine salt, containing 1.3 w/w% diclofenac hydroxyethylpyrrolidine salt) revealed superior transdermal absorbability of the preparation of the present invention (No. 2).

### (Example 2) Preparation of transdermal absorption adhesive preparation containing lidocaine

Each agent is weighed to achieve the composition (w/w%) of the following Table 2, a styrene-isoprene-styrene copolymer is added to liquid paraffin, and the mixture is dissolved by heating to about 160°C. The solution is cooled to 100°C, a solution of lidocaine in propylene glycol and polyethylene glycol 400 is added and the mixture is mixed and stirred to give an adhesive base.
The adhesive base is applied to a silicon-treated polyester film, and adjusted to an amount of 1000 g/m². A polyester non-woven fabric is laminated on the surface of the adhesive base. This was cut in a desired size to give the object transdermal absorption adhesive preparation.

**[Table 2]**

| agent | No. 3 |
|---|---|
| elastomer: styrene-isoprene-styrene copolymer | 12 |
| liquid paraffin | 78 |
| polyhydric alcohol: propylene glycol | 1 |
| polyethylene glycol 400 | 4 |
| drug: lidocaine | 5 |

The transdermal absorption preparation prepared in the above-mentioned Table 2 showed good feeling and good adhesive property to the skin, like the transdermal absorption preparation of Example 1.
As for transdermal absorbability, comparison of commercially available adhesive preparations containing lidocaine [Comparative Example 2 (containing 60 w/w% lidocaine) and Comparative Example 3 (containing 10 w/w% lidocaine)], and the present invention preparation (No. 3) has revealed that the preparation of the present invention containing lidocaine at a low concentration of 5 w/w% shows superior transdermal absorbability to Comparative Examples 2 and 3 having high concentrations.

### (Comparative Example 1) Commercially available diclofenac adhesive preparation

An adhesive preparation containing 1.3 w/w% of a diclofenac hydroxyethylpyrrolidine salt in an aqueous base composed of water, water-soluble polymer, polyhydric alcohol and the like was used. The base weight was 1000 g/m².

### (Comparative Example 2) Commercially available lidocaine adhesive preparation A

An adhesive preparation containing 60 wt% of lidocaine in an acrylic acid-octyl acrylate ester copolymer was used. The base weight was 19.6 g/m².

### (Comparative Example 3) Commercially available lidocaine adhesive preparation B

An adhesive preparation containing 10 wt% of lidocaine in a base composed of styrene-isoprene-styrene copolymer, alicyclic saturated hydrocarbon resin, liquid paraffin and the like was used. The base weight was 110 g/m².

### (Experimental Example 1) Transdermal absorbability evaluation test (A) of adhesive preparation

The abdominal skin sampling of male Wister rat (5-week-old) was set on a vertical Franz diffusion cell, and test adhesive preparations of Example 1 and Comparative Example 1 were each punched out in a circular shape (diameter 1.0 cm) and adhered to the rat skin of the diffusion cells (n=3). For the receptor, 10% ethanol saline was used, and the amount of drug that permeated the rat skin after a given time was quantified by HPLC.
The results thereof are shown in Fig. 1. As is clear from Fig. 1, the transdermal absorption preparation of the present invention showed about 4 times superior transdermal absorbability as compared to Comparative Example 1 (aqueous base).

### (Experimental Example 2) Transdermal absorbability evaluation test (B) of adhesive preparation

The abdominal skin sampling of male Wister rat (5-week-old) was set on a vertical Franz diffusion cell, and test adhesive preparations of Example 2 and Comparative Examples 2 and 3 were each punched out in a circular shape (diameter 1.77 cm) and adhered to the rat skin of the diffusion cells (n=3). For the receptor, 10% ethanol saline was used, and the amount of drug that permeated the rat skin after a given time was quantified by HPLC.
The results thereof are shown in Fig. 2. As is clear from Fig. 2, the transdermal absorption preparation of the present invention showed about 6 or more times superior transdermal absorbability as compared to commercially available lidocaine products.

### Industrial Applicability

The transdermal absorption preparation of the present invention is superior in the feeling on adhesion to the skin and transdermal absorbability, and may be utilizable to the improvement of the property of the existing transdermal absorption preparations and the development of a new transdermal absorption preparation.

## Claims

1. An adhesive sheet for adhesion to the skin, which comprises a support and an adhesive layer formed on the support, wherein the adhesive layer comprises at least a thermoplastic elastomer, and more than 300 parts by weight of liquid paraffin per 100 parts by weight of the elastomer, and wherein the adhesive layer comprises not more than 10 wt% of a tackifier.

2. The adhesive sheet according to claim 1, wherein the content of the liquid paraffin in the adhesive layer is not less than 60 wt%.

3. The adhesive sheet according to claim 1 or 2, wherein the thermoplastic elastomer is a styrene-based block copolymer.

4. The adhesive sheet according to claim 3, wherein the styrene-based block copolymer is a styrene-isoprene-styrene copolymer.

5. The adhesive sheet according to any of claims 1 - 4, wherein the adhesive layer does not contain a tackifier.

6. A transdermal absorption preparation comprising the adhesive sheet according to any of claims 1 - 5 and a drug or a pharmaceutically acceptable salt thereof in the adhesive layer of the sheet.
